# EUROPEAN PATENT APPLICATION

(11) **EP 2 385 476 A1**
(43) Date of publication of application: **09.11.2011**
(21) Application number: 11165113.9
(22) Date of filing: 06.05.2011
(51) Int. Cl.: G06F 19/00, G06Q 50/00

(54) **Method and device for handling calls in a nurse call system**

(30) Priority: 06.05.2010 US 331938 P
(71) Applicant: Televic Healthcare NV, 8870 Izegem (BE); Universiteit Gent, 9000 Gent (BE); IBBT vzw, 9050 Ledeberg (BE)
(72) Inventor: De Turck, Filip, 9550, Herzele (BE); Ongenae, Femke, 9050, Ledeberg (BE); Dhaene, Tom, 9800, Deinze (BE); Ackaert, Ann, 9041, Oostakker (BE); Verhoeve, Piet, 8930, Menen (BE); Stubbe, Brecht, 9840, De Pinte (BE)
(74) Representative: BiiP cvba

(57) **Abstract**

The present invention is related to a method for determining a priority level of a received call message in a nurse call system. The method comprises the steps of
- receiving a call message made by a patient or by a nurse for the patient or by a device for monitoring the patient,
- obtaining from a knowledge management system context-related information on the patient, said context-related information on the patient containing at least probabilistic information related to a risk profile of the patient,
- obtaining respective probabilities of the received call message belonging to various priority classes of a set of predefined priority classes, taking into account the context-related information on the patient,
- assigning to the received call message one priority class of the set based on the computed probabilities for the various priority classes.

## Description

The present invention generally relates to the field of methods and systems for healthcare communication such as patient-nurse communication.

### Background of the Invention

Information technology is widely adopted in modern medical practice, especially to support administrative tasks, electronic patient records and data management. A challenging problem today arises from the fact that several data sources and devices have to be manually combined and consulted by the staff members to take advantage of this information, even when carrying out one single task. This is a time consuming job. An underdeveloped area of solution to this problem is the use of context-aware techniques to automatically exploit the medical information available to improve continuous care and personalize healthcare. This implies an emerging demand for the integration and exploitation of the heterogeneous information available from all the wireless devices, patient records and medical data. Building context-aware applications on top of an ontology is a suitable approach to do this as has been proven in the domain of medical decision making. This approach has however not been exploited to improve the continuous care of patients. An important way to coordinate work, communicate and provide continuous care is by making use of a nurse call system.

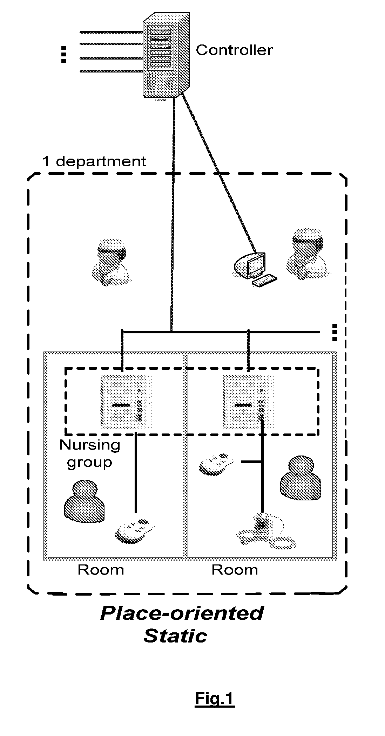

The architecture of traditional place-oriented nurse call systems is illustrated in Fig.1. Each room has at least one button which can be used by the patient to call a nurse. All the buttons in a room are connected to a *Node.* All the *Nodes* of a department are connected with each other and a *Controller.* The *Controllers* are the heart of the system. They contain the intelligence to know what must happen when a call is made, for example which nurses must be called.

The *Nodes* can be divided over different departments of the hospital or nursing home which each have their own specific settings. Within a department, the *Nodes* can be further divided into different, possibly overlapping, nursing groups. Each group can have his own configuration settings concerning for example the priorities of the different kinds of calls. Each nurse, who is identified by his or her beeper or portable phone number inside the system, is assigned to at least one nursing group. A nurse will only receive calls of the nursing groups to which that nurse is assigned to. The advantage of using nursing groups is that patients who need more attention can be equally divided amongst the groups or be put in a separate group to better distribute the workload amongst the nurses.

Traditional nurse call algorithms consist of predefined links of beeper or portable phone numbers to rooms. To make a call the patient pushes one of the fixed buttons in his room. All the beepers and portable phones of the nurses, who are in the nursing group that this room belongs to, are activated. The nurses decide on their own if they are going to interrupt their current task to answer the call or not. The nurse who reaches the room first will handle the call.

Several drawbacks and limitations of prior art solutions can be indicated. On the one hand, the current nurse call systems are place-oriented. When a patient makes a call with a button that is fixed to a wall of a room, the called nurse simply goes to the room where the call came from. Hereby two important assumptions are made: the patient must still be in the room and it must be the patient who lies in the room that made the call. A patient can also only make calls inside his room. It is dangerous to become unwell (e.g. due to heavy respiratory or heart problems) inside a hallway, staircase or outside. This leads to patients being confined to their room to ensure their safety. It may be that in some cases nurse call buttons are available in the corridors, but even then these buttons are typically at fixed positions. On the other hand, the system does not take into account various factors specific to a situation, such as the risk factors of a patient or the characteristics of the staff. Multiple nurses, namely all the nurses inside the nursing group that this room belongs to, are called. They have to decide for themselves if they are going to interrupt their current task to answer the call. They have no information about the urgency and the kind of call or about the patient to guide them in this decision. If they interrupt their current work, which can also be the handling of a call, they have to remember themselves that they have to return to it. It is possible that more than one nurse goes to answer the call. This makes the whole system somewhat unreliable and inefficient.

Overall there is a transition to a world with more mobile and wireless devices. The user friendliness and influence on nursing time has been compared of a first system comparable to the nurse call system detailed above and a second system wherein the staff members additionally were given locator badges through which they could be constantly tracked. 80% of the participants in the study preferred the second system to the first one. This is because a lot of time in a hospital is spent on *trying to find someone.* By using the locator badges this became an easier and less time-consuming task. Thus in the future, there will be an evolution to a mobile button not only for the staff members but also for each patient so that the patients can walk around freely and still make calls. This evolution implies a lot of changes, for example the nurse has to go to the exact location of the patient and the patients can make calls from anywhere in or outside the hospital.

Context information becomes increasingly important in a world with more and more wireless devices that have to be in touch with the environment around them. Lots of problems in current nurse call systems are caused by the fact that they do not take the context information into account. Nurses are often called for tasks that could also be done by a less qualified staff member. Rerouting these kinds of calls to other staff members might greatly improve response time and patient satisfaction. Studies have also shown that a large amount of calls are accidental calls. Finding a way to indicate these calls might greatly improve the work pressure put on nurses and caregivers. Some features of the nurse call system which were identified as favourable to the performance of the staff are: locating staff, direct room-to-room communication and identification of the importance of calls, e.g. accidental or not, or specifying condition and history of the patient.

Consequently, there is a need for a solution to identify the priority level of calls, so that a better distinction between e.g. accidental calls and important calls can be achieved.

There is also a need for a nurse call system that supports the transition to mobile and wireless nurse call buttons and that employs an intelligent nurse call algorithm taking the profiles of staff members and patient into account.

### Aims of the invention

The present invention aims to provide a method that allows determining the priority of a call in a nurse call system. It further aims to provide a way to assign a best fitting staff member to handle a certain call. In another aspect the invention aims to provide a controller device for use in a nurse call system that is capable of carrying out the method. The invention also aims to provide a nurse call system comprising such a controller device.

### Summary

The present invention relates to a method for determining a priority level of a received call message in a nurse call system. The method comprises the steps of
- receiving a call message made by a patient or by a nurse for said patient or by a device for monitoring said patient,
- obtaining from a knowledge management system context-related information on said patient, said context-related information on said patient containing at least probabilistic information related to a risk profile of said patient,
- obtaining respective probabilities of said received call message belonging to various priority classes of a set of predefined priority classes, based on said context-related information on said patient,
- assigning to the received call message one priority class of said set based on the obtained probabilities for the various priority classes.

The proposed method indeed allows for a dynamic priority assessment. Whereas in the prior art the call priority is statically defined in advance, in the invention a dynamic, probabilistic approach is followed. Context-related information on the patient is obtained from a knowledge management system wherein such information is contained. The context-related information at least comprises information on a risk profile of the patient. With risk profile is meant anything that may cause the patient to make calls of higher priority than other patients. This may include, but is not limited to, medical risk factors (such as diseases, earlier treatments, medication, ...) or behavioural factors (e.g.: aggressive, agitated, disoriented, confused, suicidal, ...). A risk profile outlines the number of risks, the type of risks and potential effects of the risks on the priority of the call. As a patient with a certain risk profile can still make calls of varying priority, this risk profile information should be modelled probabilistically. Various priority classes have been predefined and for each of the priority classes a probabilistic indication of the call belonging to that class is obtained based on the context-related information. This can be done by calculation (e.g. when the system is taken into use) or by using stored values (which may yield time savings as explained below). Next, given these probabilities the received call message is assigned one class of the plurality of priority classes. The selected class provides a kind of 'label' to the call which allows processing it in an adequate way.

In a preferred embodiment not only context-related information on the patient is obtained but also on a plurality of staff members. This staff related context information is then exploited to determine the staff member of the plurality of staff members that is best fitted to handle the call. This determination is based both on the priority class that was assigned to the call and on context-related information of the staff members. This context-related information may for example comprise information on the current task a staff member is carrying out, information on the location of the staff member or information on the kind of calls a staff member is allowed to handle given his/her qualification. In an advantageous embodiment the context-related information of the staff members is organised in profiles. Determining the most suitable staff member then means achieving an optimal matching between the profiles of the various members of the staff and the profile of the patient. The call message can then be redirected to that staff member. An efficient workload distribution can so be achieved between all the staff members with similar competences, as well as a good balance between safety and cost, without undermining the quality of care.

In a preferred embodiment the method further comprises the step of determining to which class of a plurality of call classes said call message belongs to, based on context-related information on location, on the person making the call, on indications (e.g. numerical values) provided by a device that is monitoring the patient. Examples of classes to divide calls into are normal calls made for medical problems, service calls for a caring task, sanitary calls etc. Certain classes of calls that can be made, may be preserved for staff members only, e.g. urgency calls or technical assistance calls. In the subsequent steps of computing and assigning the determined class of call, e.g. normal or sanitary, is taken into account. Further the context of the call can be taken into account, e.g. elements like time of the day that the call is made, during the day or at night, ...

The step of assigning the call is preferably performed based on an algorithm that exploits threshold levels derived from a statistical analysis of the call behaviour. A set of probabilities is obtained each indicating the probability that the call has a certain priority level of the predefined set of priority levels. However, one priority must be attached to the call, so this priority can be used in the nurse call algorithm. To resolve this issue thresholds are employed. If the probabilistic value for a certain priority class is higher than or equal to the threshold for this priority class, it gets this priority.

Apart from the risk profile, the context-related information on the patient advantageously comprises information on the patient's location. This additional information is especially useful when determining the most suitable staff member to handle the call. Obviously, this information is preferably combined with location information of the staff members.

Beside location information the context-related information on the staff members advantageously comprises information on the current task each staff member is performing and/or environmental information, e.g. the priority of that current task, the location, characteristics like gender, language, ...

The knowledge management system is preferably an ontology. Although a purely rule based system may be envisaged as well, an ontology system provides clear benefits when the system grows larger, as it is easier to maintain than a rule-based system.

In a preferred embodiment certain information is precalculated and stored as known, given facts in the ontology. This improves the speed the probabilistic reasoning.

In another aspect the invention relates to a controller device for use in the method for determining a priority level as previously described. The controller device is arranged for receiving a call message made by a patient or by a nurse for the patient or by a device for monitoring the patient and for obtaining from a knowledge management system context-related information on the patient, wherein the context-related information on the patient contains at least probabilistic information related to a risk profile of the patient. The controller device is further arranged for obtaining respective probabilities of the received call message belonging to various priority classes of a set of predefined priority classes, taking into account the context-related information on the patient. The controller device is further arranged for assigning to the received call message one priority class of the set based on the computed probabilities for the various priority classes.

In an preferred embodiment the controller device is further arranged for obtaining context-related information on a plurality of staff members and for selecting a staff member of the plurality to handle the call message thereby taking into account the assigned priority class of the received call message and the context-related information on the plurality of staff members.

In yet another aspect the invention relates to a nurse call system comprising a controller device as described.

### Brief Description of the Drawings

Fig. 1 represents a prior art place-oriented nurse call system.

Fig. 2 represents a person-oriented nurse call system.

Fig. 3 represents the general concept of a platform according to the invention, wherein probabilistic risk assessment and profile management are applied.

Fig. 4 illustrates a modelling of context information about staff members and patients.

Fig. 5 illustrates a possible modelling of helper characteristics and risk factors.

Fig. 6 illustrates a modelling of context information about calls and tasks.

Fig. 7 illustrates the generation of a test group of a hospital department.

Fig. 8 represents the determination which kind of call has been made.

Fig. 9 represents an embodiment of the algorithm according to the invention to find the correct staff member to handle a call.

Fig. 10 represents an overview of the nurse call system platform.

### Detailed Description of the Invention

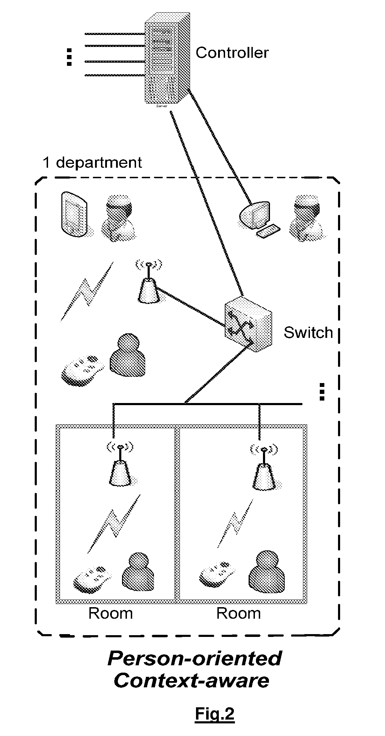

Whereas in the prior art place-oriented nurse call systems are described, the present invention is concerned with a person-oriented approach of a nurse call system. Probabilistic reasoning is exploited in an algorithm to determine the priority of a call based on the risk profile of the patient. This call priority determination greatly helps in efficiently dealing with accidental calls and calls intended for staff members with another qualification. Further the priority of a call is used along with context-related information on the staff members in an algorithm to determine the most suitable staff member, whereby the algorithm is capable of dynamically adapting to the situation at hand. The proposed invention also supports the transition to mobile and wireless nurse call buttons in hospitals and nursing homes. Fig.2 illustrates the person-oriented and context-aware approach, as opposed to the traditional place-oriented solution presented in Fig.1.

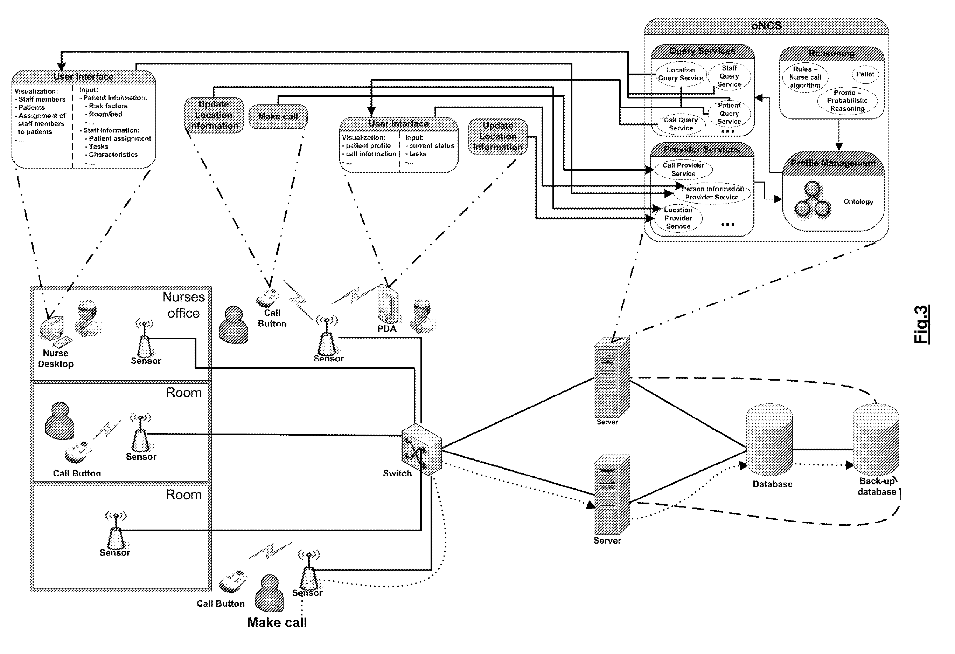

Patients can walk around freely in the hospital with their wireless nurse call buttons. These buttons periodically broadcast a message which is picked up by the nearby sensors. A large number of available sensors guarantees that another sensor can pick up the message in case the closest one is malfunctioning. This information then travels through the switch to the back-end server (i.e. the controller device), as can be seen in the bottom part of Fig.3. Existing state-of-the-art algorithms can be used to detect the accurate location of the patient out of this information by taking, for example, the signal strength perceived by the various sensors into account. When the location cannot be calculated or is inaccurate, the previous location information is used until the next broadcast is detected. When the patient makes a call, a call message is sent in a similar manner. In this case the controller device does not only update the location of the patient, but also initiates the algorithm to find the most appropriate staff member to handle the call. The location of the patient is updated and monitored until a staff member is at the scene to handle the call.

Each staff member has a communication device like e.g. a PDA which provides a transparent and user-friendly *Graphical User Interface* (*GUI*). Information about the patients such as their risk profile or location can be requested. The PDA also notifies the staff member of calls that this staff member is assigned to. The staff member is able to request more information about the call such as information about the person who made it, where it originated from and what the priority is. The staff member can also indicate if he/she is going to handle the call or not. The sensor network is used to automatically detect that the staff member is at the location of the patient and is thus handling the call. Alternatively, the staff member can launch a signal allowing location determination.

A desktop is available in each department which provides the head nurse with a *GUI* to input and visualize information about the department. The head nurse can input information about the patients, such as their risk factors or which rooms they occupy, and about the staff members, such as their characteristics or the patients they are responsible for. Information about the department is displayed in an overview window which allows the head nurse to view which nurse has been assigned to which patient and where all the staff members and patients currently are. By clicking on a staff member or patient, the head nurse can view additional information about this person.

The software platform of the nurse call system transparently handles all the communication to and from these devices. In order to ensure reliability and scalability it preferably runs on multiple servers (i.e. multiple controller devices). When a server goes down, another server can still process all the requests. Standard load-balancing algorithms can also be used to distribute the requests amongst the different servers.

A rule-based system can be used to model a dynamic nurse call algorithm that takes into account the context information contained in a database. However, such a rule-based system has the tendency to become very complicated, making it difficult for domain experts to keep track of how individual rules contribute to solving the problem and to predict how the different rules will interact. The individual rules can become very complex and the interactions between the different rules make it difficult to guarantee the consistency of the rule set. This is because the rules encode both the domain knowledge and the problem-solving logic. This means that changing the rules modifies both the domain knowledge and the problem-solving behaviour.

In an advantageous embodiment of the invention, however, an ontology is used to model all the profile information about the patients, staff members and context-information about the call. Ontologies can be used to structure and represent the knowledge about a domain in a formal way. An ontology represents the concepts within this domain, the relations and their attributes. This knowledge can then be shared and reused. Because of the foundation of ontologies in First-Order Logic (FOL), the models and description of the data in these models can be formally proven. It can also be used to detect inconsistencies in the model as well as infer new information out of the correlation of this data. This proving and classification process is referred to as reasoning. In the present invention the ontology contains all the necessary context information about the hospital such as information about the profiles of the staff members, the profiles of the patients and the calls. It also contains information about the risk factors of the patients. This way, the ontology is used to model the domain knowledge, while rules are used to model the problem-solving behaviour using the context information obtained from the ontology.

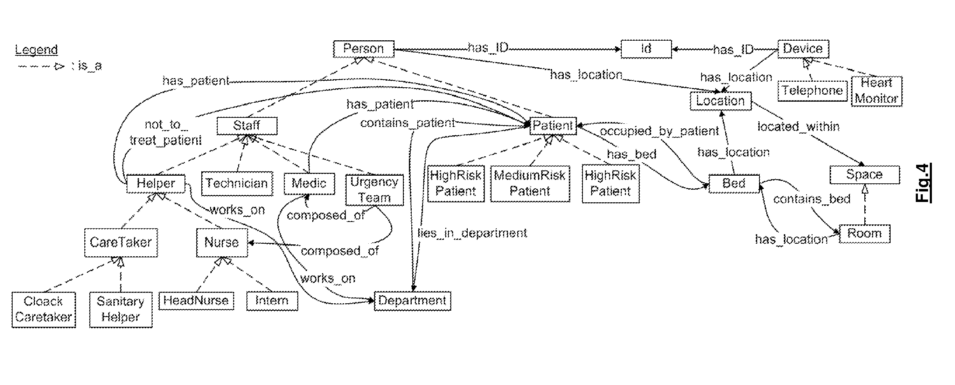

From field trials and experiments it was derived which context information was relevant. The context information on patients and the staff members of the hospital who can answer and make calls is modelled. One possible way of doing so is shown in Fig.4. The current location is tracked for each staff member and patient. All staff members have associated beepers and/or portable phone numbers. It is also modelled on which departments a staff member works and on which department a patient lies. Some information is also maintained for administrative purposes such as names, IDs, beds and rooms. Helpers can have different specializations. For example, the following two special types of nurses can be defined: head nurses and interns. Special groups of caretakers can also be defined. Sanitary helpers are responsible for *caring* tasks such as cleaning a bed or fluffing a pillow. Family caregivers are volunteers who can also be called for *caring* tasks.

Whereas in the place-oriented system, each helper was associated with a nursing group, it is more logical in the person-oriented system to associate each helper with a group of patients for whom this helper is responsible. This makes the system very flexible, as these groups can be dynamically adapted to equally divide the work load among the different helpers. Each medical staff member is also responsible for one or more patients.

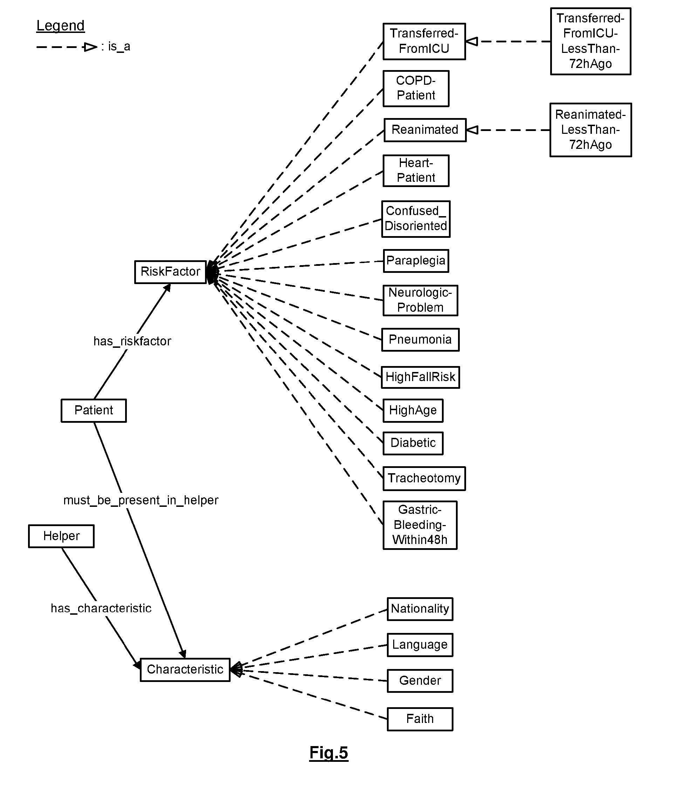

Some characteristics about the helpers are modelled, which can be seen in Fig.5. In the shown example the following classes are used: which languages the helpers speak, their gender, their nationality and their religious beliefs. Helpers can indicate patients that they do not want to treat e.g. because it is a family member. Patients can then indicate which characteristics they would prefer to be present in the helper that treats them. As a consequence, patients cannot directly indicate that they do not want to be treated by a particular helper.

It is indicated if a patient has one or more risk factors. A complete list of risk factors may be constructed based on a thorough study of the risk factors of patients and the reasons for the calls that they make. Fig.5 shows by way of example a (non-exhaustive) list of risk factors was assembled by experts from both the medical and nurse call domain.

When a patient exhibits a risk factor, he is assigned a probability of belonging to a risk group namely High, Medium and Low Risk Patients. The assigned probability may also be influenced by the department where the patient resides. An example of a set of probabilities, as determined by domain experts, can be seen in Table 1. As it is difficult to determine exact probabilities for these cases, probabilistic intervals are preferably employed. For example, a diabetic patient has at least 50% chance of being a high risk patient. This is encoded as the probabilistic interval [0.5,1]. Note that the probability interval [1,0] in the table means that this combination is not possible. In other words, a patient with this risk factor never belongs to this risk group.

**Table 1**

| **Patient has risk factor:** | **High Risk** | **Medium Risk** | **Low Risk** |
|---|---|---|---|
| Elderly (high age) | [0,0.2] | [0.5,1] | [0,0.3] |
| Diabetes | [0.5,1] | [0,0.3] | [0,0.2] |
| Heart patient | [0.5,1] | [0,0.4] | [0,0.1] |
| High fall risk | [0.7,1] | [0,0.2] | [0,0.1] |
| Neurological problem | [0.5,1] | [0,0.3] | [0,0.2] |
| Tracheotomy | [0.8,1] | [0,0.2] | [1,0] |
| COPD patient | [0.6,1] | [0,0.3] | [0,0.1] |
| Paraplegic | [0.6,1] | [0,0.3] | [0,0.1] |
| Pneumonia | [0.5,1] | [0,0.4] | [0,0.1] |
| Gastric bleeding within 48 hours | [0.8,1] | [0,0.2] | [1,0] |
| Transferred from the ICU | [0.6,1] | [0,0.4] | [1,0] |
| Transferred from the ICU within 72 hours | [0.7,1] | [0,0.3] | [1,0] |
| Reanimated | [0.6,1] | [0,0.4] | [1,0] |
| Reanimated within 72 hours | [0.8,1] | [0,0.2] | [1,0] |
| Confused or disoriented | [0.6,1] | [0,0.3] | [0,0.1] |

Patients can of course have several risk factors. In this case, the system reasons over the different probabilities to determine the general probability that a patient belongs to a risk group. This reasoning process is explained more in detail below. It is possible to override these general probabilistic assignments by adding a statement that this particular patient belongs to a risk group with 100% probability (this is probability interval [1,1]).

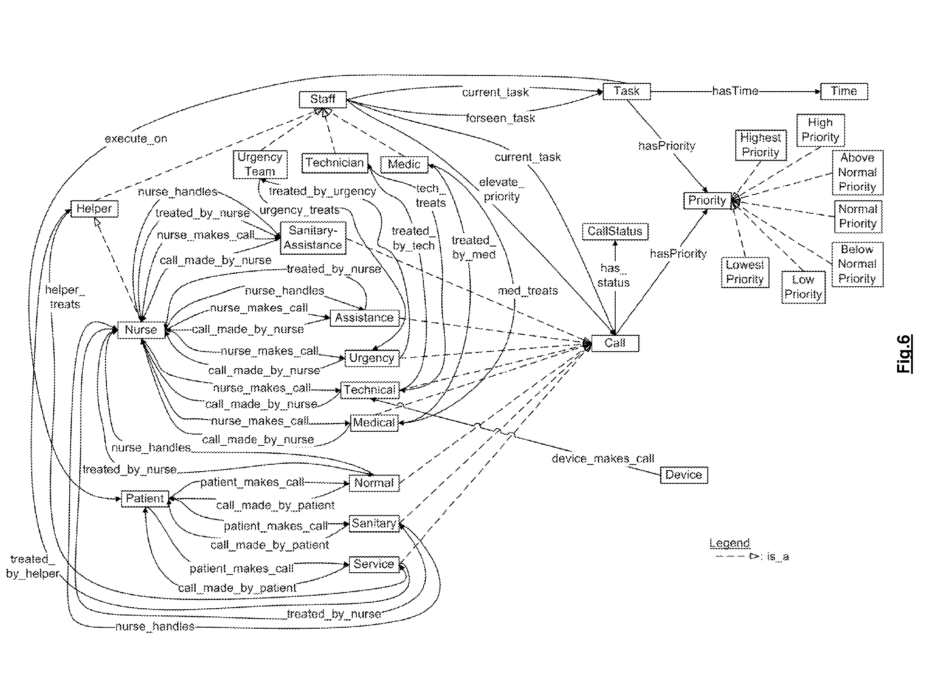

Each staff member has an associated current task, as can be seen in Fig.6. For each staff member, it is logged if this staff member is free or busy. Staff members can be handling a call or doing other tasks, e.g. giving medication to a patient or restocking. For each task the time by which the task should be completed can be indicated. The patient for whom this task should be done can also be attached. It is also possible to maintain a list of tasks that a staff member should complete. A task can also be assigned a priority.

Information is maintained that is applicable to each call such as the sequence number, the start and end time and the persons who made and handled the call. Each kind of call also has a time-out time. A call can have different statuses. When a call is launched, it has the status Active. This status changes to Answered when a staff member has been called. While the staff member is treating the call, the status changes to Busy. When the job is completely finished, the status is set to Finished.

For the different kinds of calls that can be made, information is modelled that pertains to the specific type of call. For each call it is indicated which kind of person can make the call. As can be seen in Fig.6, three kinds of calls can be launched by patients. A normal call is made for medical problems and a service call is made for a "caring" task. When a normal call is made inside a sanitary room the call is automatically transformed to a sanitary call. All the other calls, namely urgency, medical, technical and (sanitary) assistance calls, are launched by nurses. When an assistance call is made inside a sanitary room it automatically becomes a sanitary assistance call. Which kind of staff member can answer the call is also maintained, e.g. urgency calls must be handled by an urgency team.

The different devices that can be present inside a hospital also may be taken into account. Devices such as heart monitors are able to launch technical calls when, for example, their cable is unplugged.

The probabilistic assignment of patients to risk groups is used to determine the priority of the calls made by these patient and the calls made by nurses for these patients. In the example given here there are seven classes of priorities: Highest, High, Above Normal, Normal, Below Normal, Low and Lowest priority as is illustrated in the upper right corner of Fig.6. The priority of a call is also based on its kind e.g. normal or sanitary. So when a patient from a risk group, makes a certain kind of call, this call is assigned a probability of having a certain priority. For example, when a high risk patient makes a normal call, this call has 2% chance of having a high priority. For the purpose of this example these probabilities are determined as shown in Table 2 and Table 3. Combinations with probability interval [1,0] (i.e. combinations that cannot occur) are depicted with an empty place in the table to prevent cluttering. Strict probabilities were used here, but they can easily be translated to intervals by using equal upper and lower limits. For example, probability 0.2 becomes probability interval [0.2,0.2]. Note that urgency, medical and technical calls generally get the highest, low and lowest priority respectively. Thus, the priority of these kinds of calls does generally not depend on the risk factors of the patient.

**Table 2**

| **Priority:** | | **Highest** | **High** | **Above Normal** | **Normal** | **Below Normal** | **Low** | **Lowest** |
|---|---|---|---|---|---|---|---|---|
| High risk patient makes a | Normal call | | 0.2 | 0.6 | 0.2 | | | |
| | Sanitary call | | 0.3 | 0.6 | 0.1 | | | |
| | Service call | | | 0.2 | 0.2 | 0.6 | | |
| Medium risk patient makes a | Normal call | | | 0.3 | 0.6 | 0.1 | | |
| | Sanitary call | | | 0.4 | 0.5 | 0.1 | | |
| | Service call | | | | 0.2 | 0.4 | 0.4 | |
| Low risk patient makes a | Normal call | | | | 0.6 | 0.3 | 0.1 | |
| | Sanitary call | | | | 0.7 | 0.2 | 0.1 | |
| | Service call | | | | | 0.4 | 0.4 | 0.2 |

**Table 3**

| **Priority:** | | **Highest** | **High** | **Above Normal** | **Norma l** | **Below Normal** | **Low** | **Low est** |
|---|---|---|---|---|---|---|---|---|
| Nurse treating high risk patient makes a | Assistance call | 0.2 | 0.5 | 0.3 | | | | |
| | Sanitary Assistance call | 0.3 | 0.5 | 0.2 | | | | |
| Nurse treating medium risk patient makes a | Assistance call | | 0.6 | 0.3 | 0.1 | | | |
| | Sanitary Assistance call | | 0.7 | 0.2 | 0.1 | | | |
| Nurse treating low risk patient makes a | Assistance call | | | 0.7 | 0.2 | 0.1 | | |
| | Sanitary Assistance call | | | 0.8 | 0.1 | 0.1 | | |
| Nurse makes a | Urgency call | 1.0 | | | | | | |
| Nurse makes a | Medical call | | | | | | 1.0 | |
| Nurse or device makes a | Technical call | | | | | | | 1.0 |

An algorithm is now described to reason with the probabilistic information to assign a more informed priority to a call based on the risk profile of a patient. The general probabilistic information about the assignment of patients to risk groups and the priorities of calls is exploited to determine the priority of a specific call made by a specific patient. For this the platform needs to reason about the general probabilistic information in the ontology and apply it to the situation at hand.

The probability that a specific call made by a specific patient has a certain priority can be determined by performing probabilistic reasoning. Various methods have been proposed in the literature to represent and reason about probabilistic knowledge in ontologies. On the one hand, approaches have been proposed based on combining some form of *Bayesian network theory* with an ontology. On the other hand, a probabilistic extension of *Description Logics* (DLs) has been proposed in "Probabilistic Description Logics for the Semantic Web" (T. Lukasiewicz, INFSYS Research Report, 2007).
All these techniques have their own advantages and disadvantages. The latter method, based on a probabilistic extension of *DLs,* is easy to use and understand and offers a wide range of reasoning support. All the reasoning is done in a totally logical way without any implicit or explicit translation of the *Knowledge Base* to for example a *Bayesian network.* The methods, based on some form of *Bayesian network theory,* are more expressive and can express very difficult probabilistic dependencies. However, these methods have the limitation that both a traditional ontology and a probabilistic model have to be maintained. Logical and probabilistic reasoning has to be done separately. Because of these disadvantages and because the uncertainties needed for this use case can be expressed by the second approach, namely the probabilistic extension of DLs, this approach is advantageously chosen as method to represent and reason with probabilistic knowledge in ontologies. An open source implementation of this method, called Pronto, can for example be used (see "Pronto: A Non-monotonic Probabilistic Description Logic Reasoner", P.Klinov, The Semantic Web: Research and Applications. ESWC 2008: Proceedings of the 5th European Semantic Web Conference, June 1-5 2008, Tenerife, pp. 822-826).

By using the previously described probabilistic algorithms, one can determine the probability that a specific call made by a specific patient has a certain priority. For each of the seven possible priorities, the probability that the call has this priority is available, by actually calculating it or by retrieving it from storage if it was already calculated before. However, one priority must be attached to the call, so that this priority can be used in the nurse call algorithm as explained below. More details on the threshold algorithm to realize this are given in the next paragraph.

To determine the suitable priority class for the call based on the probabilistic values thresholds are employed. If the probabilistic value for a certain priority class is higher than or equal to the threshold for this priority class, it gets this priority. Thus these thresholds need to be determined, based on specific characteristics, e.g. number of calls, needs and preferences of the department or hospital.

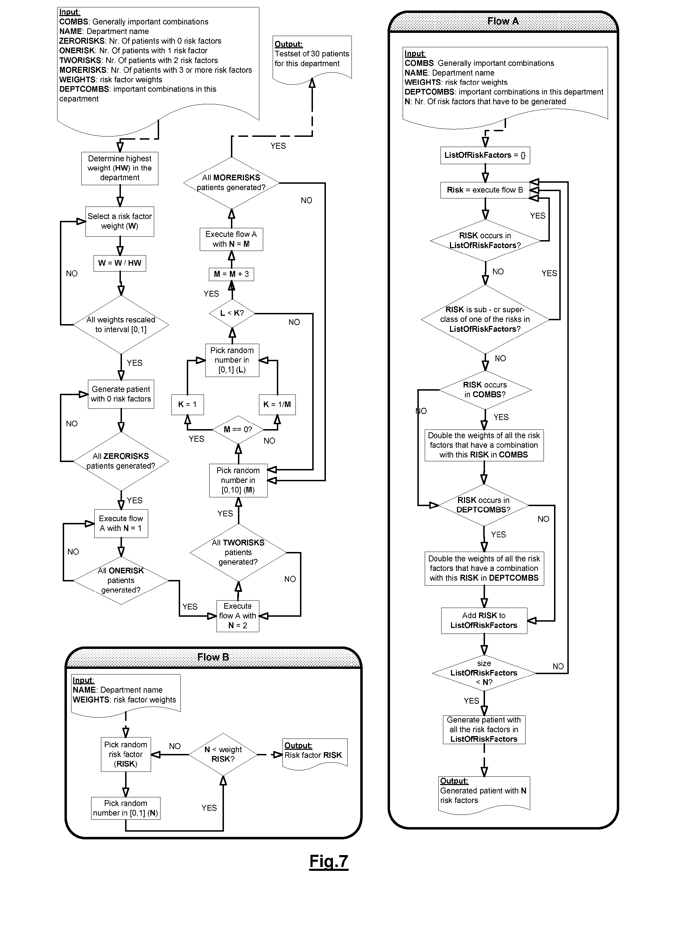

The thresholds for each priority class for a particular department can for example be determined by performing simulations of calls. The risk profile of the patients within this department is determined and weights are assigned to these risk factors that reflect how frequently they occur in this department. Combinations of risk factors that are deemed to be more frequent than others are also specified. For example, the risk factors *neurological problem* and *disoriented*/*confused* often occur together for example in patients with multiple sclerosis. In some hospital departments certain combinations may be encountered more frequently than in other departments. In an illustrative example, based on the collected data about the risk profiles of the patients in a department, 20 test groups and 10 validation groups of patients with risk factors are randomly generated. Each group contains as many patients as there are beds within the department. The algorithm used for this random generation is visualized in Fig. 7. Note that Flow B takes the possible combinations into account. If a risk factor is chosen then all the weights of the risk factors that it can combine with, are doubled. Consequently, these risk factors, and thus these combinations, have more chance of being chosen. Note that it is possible that a weight of a risk factor is doubled twice if it occurs both in the general and department specific combinations. For the patients with three or more risk factors, one first needs to determine how many risk factors are going to be generated. As can be seen in the left upper corner of Fig. 7, a weighted procedure was used to make sure that a low number of risk factors is more plausible than a high number of risk factors.
To determine the thresholds, each of the generated patients makes each kind of call once. For each call, the probabilistic intervals are calculated for each of the seven possible priorities using the probabilistic reasoning algorithms. These intervals indicate for each priority category the probability that this call has this priority. For each priority category only a limited number of probabilistic values can be obtained for the lower bound of the interval. These lower bounds are considered as possible thresholds.

For each combination of thresholds represented by the lower bounds of the calculated probabilistic intervals, it is determined for each call which priority category it gets. If the call has a probabilistic interval for the highest priority for which the lower bound is higher than or equal to the threshold for the highest priority class, the call gets the highest priority. If not, the same condition is checked for High, Above Normal, Below Normal, Normal, Low and Lowest priority classes and thresholds. If none of these conditions hold, the call gets the status Undetermined. The Below Normal threshold is checked before the Normal threshold to ensure that the default class that calls are assigned to is the Normal priority class. The Low and Lowest priority classes are generally reserved for technical and medical calls and should thus not be assigned often to other kinds of calls.
Using the above algorithm, the percentage of calls that are assigned to each priority category and the percentage of 'Undetermined' calls is calculated for each combination of thresholds. A curve fitting algorithm is used to determine the appropriate combination of thresholds for this department. The combination of thresholds is searched for which the percentage deviates least from the ideal distribution. Preference is given to combinations with the least amount of 'Undetermined' calls. The ideal distribution is determined based on the characteristics of the department, e.g. frequency of calls. For example the following distribution reflects a realistic hospital environment: 5% calls with Highest priority, 10% with High priority, 25% with Above Normal priority, 35% with Normal priority, 25% with Below Normal priority, with 0% Low priority and 0% with the Lowest priority. The tested kinds of calls generally do not have the Low or Lowest priority as these categories are preserved for medical and technical calls. The middle categories, namely Above Normal, Normal and Below Normal, generally contain more calls as most calls are made for simple requests.

Attention has to be paid to the scalability of the system. When more and more probabilistic statements are added to the ontology, the system should still be capable of handling them in a reasonable time. To speed up the probabilistic reasoning the following optimisation can be applied. First, during down-time, the probabilistic values indicating that this patient is a high, medium or low risk patient are calculated and stored as known facts in the ontology. This does not have to be repeated often, as risk factors do not change a lot during a patient's stay in the hospital. Next, when a call is made, all the probabilistic statements needed to calculate the priority of this call are extracted from the ontology. Each time, the statements about the probabilistic assignment of this patient to the risk groups and the statements about the generic probabilistic assignment of this kind of call to the priority groups are extracted. For example, there may be 3 statements about risk groups and 9 about the assignment of the kind of call to the priority groups, hence at most 12 statements in total.

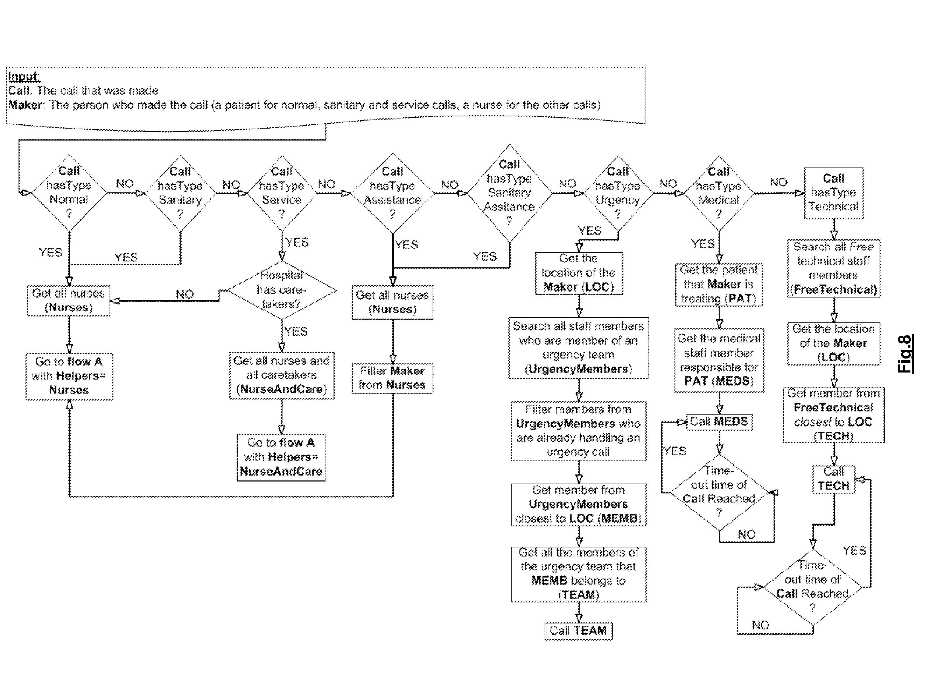

An algorithm is now proposed to find the correct staff member to handle a call. It uses the information stored in the ontology. It first determines which kind of calls has been made as illustrated in the flow chart of Fig.8.

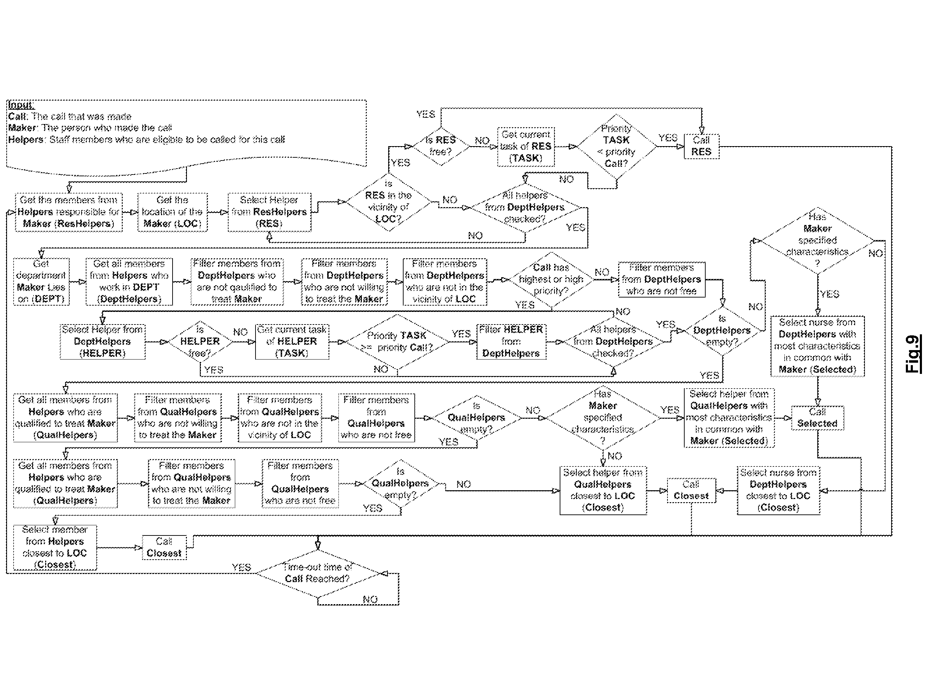

Normal, sanitary, service and (sanitary) assistance calls employ the same basic algorithm which is visualized in Fig.9. The difference is that for normal, sanitary and (sanitary) assistance calls only nurses can be called. For service calls caretakers can also be called. It is also made sure that the nurse that made the (sanitary) assistance call, cannot be called to answer this call.

The algorithm first checks if the responsible nurse or caretaker can be called. Note that this responsible staff member can also be called if he/she is busy with a task that has a lower priority than the current call. If the responsible nurse or caretaker cannot be called, all the helpers who work on the department where the patient who the call is for lies, are investigated. It is assumed that a nurse, who works on a department where the patient lies, has more background information about the illnesses and concerns of this patient. Only for calls with the highest or high priority helpers are considered that are busy with a task with a lower priority. Otherwise these helpers will never be able to finish the work for the patients they are responsible for. If this option still does not offer a solution, the search is widened beyond the scope of the department and the helpers in the whole hospital are taken into account. If the result is empty again, this means that there are no available nurses in the direct vicinity. The distance becomes a deciding factor at this moment, so the closest nurse with right properties is selected, e.g. free, willing and qualified. If this still does not offer a solution, all the nurses in the hospital are considered and the one who is closest to the patient is called. Note that the characteristics are only used to choose among different available nurses. They are never used to decide that a nurse cannot handle a patient.

The algorithm has a time-out procedure. If a staff member has not indicated that he/she is going to handle the call within the time-out time that is specified for this type of call in the ontology, another staff member is selected to handle the call by running the algorithm again.

Urgency, medical and technical calls each have their own algorithm as can be seen in Fig. 8. For urgency calls, the priority lies on finding a person who is near instead of a person who is free. This is necessary because lives are at stake when an urgency call is issued. A time-out procedure is not needed here, as an urgency call will always be immediately answered. The algorithms for the technical and medical calls are rather simple and straightforward because they generally have a very low priority.

Note that a staff member can sometimes be called while he/she is already busy with a task. It is up to the staff member to decide if he/she is going to interrupt his/her current task or not. In contradiction to the place-oriented case, the staff member knows that the new call has a higher priority than the task that this staff member is currently working on. Based on these priorities the staff members can make a more founded decision to interrupt their current task or not. If the staff member decides to answer the new call, the system automatically interrupts the current task of this staff member. If the task is a call, another staff member is searched to handle the call. If it is not a call, the task is added to the list of tasks that this staff member must do. So the staff member does not have to remember himself that he/she has to return to a task or that he/she has to call some other staff member.

As already mentioned before, a preferred embodiment of this invention employs an ontology. Some more details are now provided on an implementation with an ontology.

Different languages exist to digitize an ontology. The *Ontology Web Language (OWL)* is advantageously chosen for a number of reasons. First, *OWL* is a recommendation by the World Wide Web Consortium (W3C) and is the most widely used and well-known ontology language. Secondly, using one of the three sublanguage flavours of *OWL, OWL-Lite, OWL-DL* and *OWL-Full,* one can easily adapt to the required expressiveness at hand. *OWL-DL* is based on *Description Logics,* a decidable part of *First Order Logic.* This ensures that reasoning on *OWL-DL* models is computationally complete and decidable, which means that all computations will end in finite time. Thirdly, there also exist a wide range of tools for *OWL* such as editors and visualization tools. Sophisticated *Reasoners* exist that allow checking the consistency of the language, classifying the ontology and so on. *OWL* can also easily be integrated with different *Rule* platforms. This allows expressing *Rules* on top of the ontological model. *OWL* can be queried with *SPARQL.* Moreover, *OWL* is the only ontology language for which there exist mature tools to express and reason about probabilistic knowledge.

The nurse call system platform is built as an extension of the *Context-Aware Service Platform (CASP),* as described in the paper *"*Design of CASP: an open enabling platform for context aware office and city services" (M. Strobbe, et al., Proc. MUCS2007, Munich, pp. 123-142, May 2007). The *CASP framework* is a collection of bundles for *OSGi* to handle context information. The *OSGi Framework* is an open service platform for the delivery and control of different applications and services to a certain type of networked device in the environment. In this case the devices are the portable nurse call buttons, the sensor nodes, the PDAs and the nurse desktop. The *OSGi* framework can best be seen as a container that can host various applications, which are called bundles in *OSGi.* It is possible to plug new bundles into the *OSGi framework* at any time. This expands the framework with new possibilities and services. These new services can be dynamically discovered by the other bundles. So basically, *OSGi* technology provides the standardized primitives that allow applications to be constructed from small, reusable and collaborative components. The advantages of the *OSGi framework* for the implementation of the nurse call system are:
- The life cycle of a bundle can be controlled from anywhere in the network.
- Bundles can be removed, changed and added on the fly without having to interrupt the work of other bundles. This is very important in an application where a minimal level of service is critical. New bundles could be tested and changed without having to shut down the entire system.
- To minimize the coupling, as well as making these couplings manageable, the bundles can dynamically discover and use each other's services. In an environment with a lot of wireless devices this is a very important feature.
- It is *Java* platform based so it has advantages such as portability and object orientation.

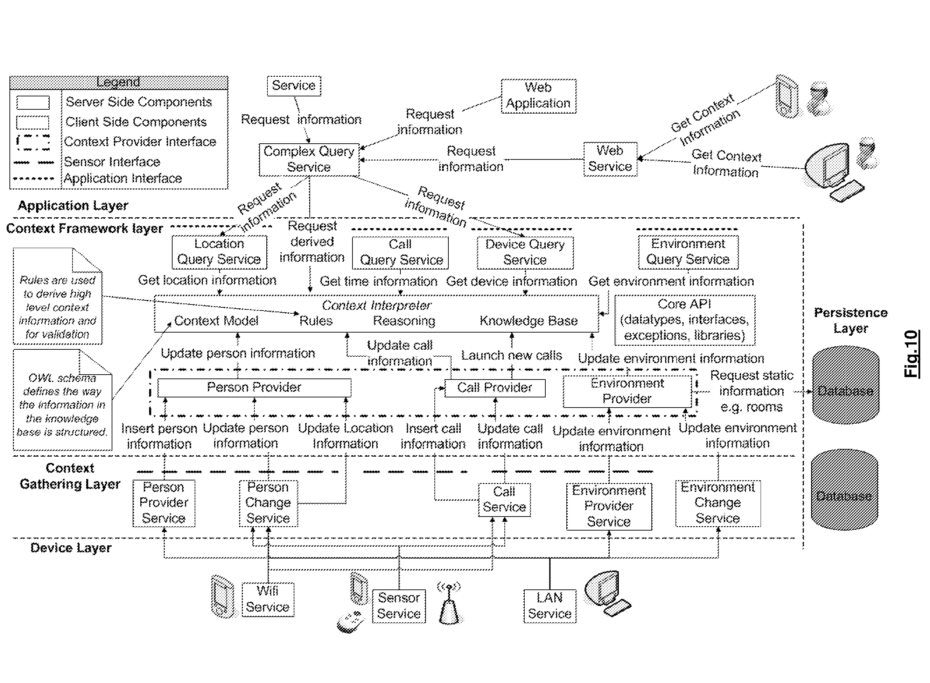

An overview of the nurse call system platform is shown in Fig.10. The *Context Framework Layer* is the most important layer. Within this layer the *Context Interpreter* controls all the context information. The ontology determines the structure of the *Knowledge Base.* The *Knowledge Base* contains all the data that conforms to the ontology. The *Context Model* provides access to the ontology. The layer also holds all the *Rules* that work with the information in the *Knowledge Base.*

The different *Context Providers* allow importing external information into the framework. This information is then added to the *Knowledge Base.* For example, the *Person Provider* is used by the sensor nodes to insert new information about the location of the patients and staff members into the *Knowledge Base.* This new information can come from a database (*Persistence Layer*) or directly from a device (*Device Layer* and *Context Gathering Layer*). Currently three *Context Providers* are provided: the Person Provider, the Environment Provider and the Call Provider. All the *Context Providers* implement a common interface, namely ContextProvider, which makes it easy to plug new *Context Providers* into the framework.

The *Query Services* are used to extract information from the *Knowledge Base.* This ensures that application developers do not have to write the error-prone queries themselves. They neither have to translate the results of the queries to usable *Java*-objects. The *Query Services* can be used to visualize the knowledge or to use the information in another application (*Application Layer*).

The methods in the *Context Providers* and *Query Services* are implemented by using *SPARQL* which is a language to query ontologies. *SPARQL-*queries are used to collect the necessary information from the *Knowledge Base.* The *Context Providers* then use this information to either adapt or create new information in the ontology. So in short, the *Context* Providers translate the provided information to *OWL* constructs which are added to the *Knowledge Base.* The *Query Services* on the other hand use the queried information to create Java-objects which can be returned to the user or application. So basically, the *Query Services* translate the *OWL* constructs from the *Knowledge Base* to *Java*-objects.

To make the platform more generic some *Web Services* were developed. These *Web Services* allow applications and devices from anywhere in the network to call methods to add new information to the *Knowledge Base,* such as making new patients, nurses or calls, or extract information such which nurse has been called to answer a call. These methods call the *Context Providers* and *Query Services* to add or extract the knowledge from the *Knowledge Base.*

Note that the framework is modularly divided into bundles. These bundles can be plugged into the *Knopflerfish* (*OSGi*) framework and can dynamically discover each other.

The proposed nurse call algorithm is implemented using *Rules.* The *Rules* are activated when an event occurs in the *Knowledge Base* for example when a new call is added. When the condition is fulfilled, the *Rule* calls a *functor.* A *functor* does some calculations with the parameters it receives from the *Rule,* for example the new call. The *functor* can also change the information in the *Knowledge Base.*

Every kind of call that can occur is handled by a different *Rule.* For example, the following code fragment
[insert_nurse_normalcall:
(?x rdf:type ncs:Normal)
(?x ncs:has_status ?CallStatus)
(?CallStatus ncs:Kind 'Active')
novalue(?x ncs:treated_by_nurse)
->findHelper(?x)]
shows the *Rule* that reacts to a normal call. This *Rule* is activated when a normal call is launched (its status is Active and no staff member has been called to answer the call). If the condition is fulfilled the *functor* findHelper() is called which takes the call as argument. The *functor* follows the earlier described algorithm to find a correct staff member to handle the call. It adds the information that this particular staff member has to handle this particular call to *Knowledge Base* (the *treated_by* relation in the ontology). This guarantees that the *Rule* is not fired again, because the *noValue* condition is no longer fulfilled. All the other types of calls are handled in a similar manner.

*Rules* were also constructed that trigger when the status of a call is changed. The *Rules* adapt the Knowledge base for example to indicate that a nurse is busy with a call, has finished a call, the time at which the call was finished and so on. Most importantly these *Rules* also automatically interrupt the current task (if any) of the called staff member.

A last set of *Rules* is used to implement the time-out procedure for each kind of call. The following code fragment shows the *Rule* that reacts to the time-out of a normal call.
[relaunch_normalcall_timeout:
(?x rdf:type ncs:Normal)
(?x ncs:treated_by_nurse ?y)
(?x ncs:has_status ?CallStatus)
(?CallStatus ncs:Kind 'Active')
->relaunchCall(?x)]

The most important features and benefits of the proposed invention can be summarized as follows.
- *Profile management* In order to achieve a nurse call algorithm that adapts to the situation at hand, context information about the profiles of patients and staff members is managed efficiently.
- *Dynamic priority assessment:* Instead of statically defining the priority of a call in advance, it here depends on the profile of the patient and more specifically on his or her risk factors. As patients with a certain profile can still make calls of varying priority, this information is modelled probabilistically. As it is difficult to accurately determine the exact probability with which a patient with a certain profile will make a call of a certain priority, the platform is able to handle probabilistic intervals.
- *Mobile:* The platform gives the patients enough mobility. They are able to wander around the whole hospital and a limited area outside of the hospital for example the smoking area and the parking lot. They are able to make calls in all these areas without their call getting lost because of bad reception. The mobile buttons are also easy to operate.
- *Location-Awareness:* The platform is able to detect the locations of patients and staff members in a sufficiently accurate way and take this information into account when finding a suitable staff member to handle a call. This data is constantly monitored and transparently delivered to the system.
- *Efficient staff assignment:* The nurse call algorithm ensures that an optimal matching is achieved between the profiles of the staff members and the profile of the patient, when finding a suitable staff member to handle a call. An efficient workload distribution is achieved between all the staff members who can handle each type of calls. A good balance between safety and cost is achieved. The quality of care may not be undermined.
- *Reliability:* Four kinds of faults can occur: the controller device can go down, a call is not delivered to the controller device, a call is not delivered to the PDA of the staff member or the location information cannot be received or is inaccurate. The platform is able to cope with each of these situations. Calls may never be lost and it should always be able to call at least one staff member. A good logging infrastructure is provided to ensure that it is always known which patients made calls, which staff members handled them and how long it took until a staff member was at the location.
- *Performance:* The performance of the platform and the algorithms is such that general guidelines can be imposed, for example, a guideline that stipulates that at least one staff member should arrive at the location of the patient within 3 minutes when an urgency call was made and within 5 minutes for other calls. As these time constrictions include walking to the patient, the time needed by the algorithm to assign a suitable staff member to a call remains negligible.
- *Generic:* new components can be plugged-in easily, independent of implementation languages, operating systems and hardware by providing generic interfaces. New applications to visualize and input information from and into the platform are easy to develop and plugged into the system.
- *Scalability:* The platform is able to handle to large amount of profile information that is available about all the staff members and patients currently in the hospital. It is also able to handle the large amount of calls that can daily enter the system.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. Method for determining a priority level of a received call message in a nurse call system, the method comprising the steps of
- receiving a call message made by a patient or by a nurse for said patient or by a device for monitoring said patient,
- obtaining from a knowledge management system context-related information on said patient, said context-related information on said patient containing at least probabilistic information related to a risk profile of said patient,
- obtaining respective probabilities of said received call message belonging to various priority classes of a set of predefined priority classes, taking into account said context-related information on said patient,
- assigning to the received call message one priority class of said set based on said obtained probabilities for the various priority classes.

2. Method for determining a priority level as in claim 1, further comprising the step of obtaining context-related information on a plurality of staff members, and the step of selecting a staff member of said plurality to handle said call message thereby taking into account the assigned priority class of said received call message and said context-related information on said plurality of staff members.

3. Method for determining a priority level as in claim 1 or 2, further comprising the step of determining to which class of a plurality of call classes said call message belongs to, based on context-related information on said call message, whereby in said computing step the determined class is taken into account.

4. Method for determining a priority level as in any of claims 1 to 3, wherein for said predefined priority classes a threshold level is determined via a weighted sum derived from said risk profile and wherein said assigning step is performed based on said threshold levels.

5. Method for determining a priority level as in any of claims 1 to 4, wherein said context-related information on said patient comprises information on said patient's location.

6. Method for determining a priority level as in any of claims 1 to 5, wherein said context-related information on said staff members comprises at least one item of {current task, location information, environmental information}.

7. Method as in claim 6, wherein said context-related information on said staff members is organised in profiles of the various staff members.

8. Method as in any of the previous claims, wherein said knowledge management system comprises an ontology.

9. Method as in any of the previous claims, comprising the step of computing and storing said probabilities in order to speed up the probabilistic reasoning.

10. Controller device for use in the method for determining a priority level as in any of claims 1 to 9, said controller device being arranged for receiving a call message made by a patient or by a nurse for said patient or by a device for monitoring said patient and for obtaining from a knowledge management system context-related information on said patient, said context-related information on said patient containing at least probabilistic information related to a risk profile of said patient, said controller device further arranged for obtaining respective probabilities of said received call message belonging to various priority classes of a set of predefined priority classes, based on said context-related information on said patient, said controller device further arranged for assigning to the received call message one priority class of said set based on the obtained probabilities for the various priority classes.

11. Controller device as in claim 10, further arranged for obtaining context-related information on a plurality of staff members and for selecting a staff member of said plurality to handle said call message thereby taking into account the assigned priority class of said received call message and said context-related information on said plurality of staff members.

12. Nurse call system comprising a controller device as in claim 10 or 11.
